# EUROPEAN PATENT SPECIFICATION

(11) **EP 3 297 677 B1**
(45) Date of publication and mention of the grant of the patent: **12.07.2023**
(21) Application number: 16733691.6
(22) Date of filing: 17.05.2016
(51) Int. Cl.: A61K 47/10, A61K 9/00, A61K 9/48, A61K 9/08, A61K 9/14, A61K 31/198

(54) **LIQUID PHARMACEUTICAL FORMULATIONS OF TETRAIODOTHYRONINE**
FLÜSSIGE PHARMAZEUTISCHE FORMULIERUNGEN AUS TETRAIODOTHYRONIN
FORMULATIONS PHARMACEUTIQUES LIQUIDES DE TÉTRAIODOTHYRONINE

(30) Priority: 19.05.2015 IT UB20150707
(43) Date of publication of application: 28.03.2018
(73) Proprietor: Altergon S.A., 6900 Lugano (CH)
(72) Inventor: DE ROSA, Mario, 6900 Lugano (CH); SCHIRALDI, Chiara, 6900 Lugano (CH)
(74) Representative: Bianchetti & Minoja with Trevisan & Cuonzo IPS SRL
(86) International application number: PCT/IB2016/052860
(87) International publication number: WO 2016/185378

(56) References cited:
- EP-A1- 2 692 340
- EP-A2- 1 291 021
- US-A- 3 808 332

## Description

The present invention relates to liquid oral pharmaceutical formulations of tetraiodothyronine (T4) with optimised bioavailability characteristics.

### State of the art

(2S)-2-Amino-3-[4-(4-hydroxy-3-iodophenoxyl)-3,5-diiodophenyl] propanoic acid C₁₅H₁₂I₃NO₄, also called T3 or liothyronine, and (2S)-2-amino-3-[4-(4-hydroxy-3,5-diiodo-oxyphenyloxy)-3,5-diiodophenyl]propanoic acid, also called T4 or tetraiodothyronine, are two important hormones synthesised in the follicles of the thyroid, a gland located in the front of the neck, near the first rings of the trachea.

The clinical treatment of hypothyroidism, namely the complex pathological condition associated with low production of thyroid hormone, is based on the administration of T4, now available in oral forms, which are the most commonly used, and intravenous forms. The usual dose ranges from 25 to 500 mcg/day. The highest doses can be used intravenously to treat myxoedema coma.

Although pure T3 is available in pill and tablet form, said molecule is seldom used nowadays to treat hypothyroidism, because the body's physiological requirements are better served by gradual, highly controlled physiological generation of T3 from T4, which takes place in the tissues. The tablets must be taken regularly, every day, but the long half-life of the medicament, about 7 days in the case of euthyroidism and 9-10 days in the case of hypothyroidism and pregnancy, prevents major problems in the event of occasional failure to comply with the daily dose.

Medicaments, foods and specific conditions can influence the absorption and/or metabolism of T4, one of the most widely prescribed medicaments in the world, which is usually marketed in a solid formulation in the form of soft capsules or tablets (*Euthyrox, Tirosint* and *levothyroxine).* To ensure adequate absorption of T4 in the bloodstream through the intestinal wall, a crucial role is played by the process of dissolution of the pharmaceutical form and gastric acidity, which enables T4 sodium salt to be converted to the more liposoluble acid form. During its transit through the gastrointestinal apparatus, the medicament is mainly absorbed in the small intestine; specifically 21% in the duodenum, 45% in the jejunum and 34% in the ileum. Absorption takes about three hours, with an initial absorption peak after one hour.

However, the absorption process can be affected by multiple factors, such as food and medicament intake.

All foods can interfere with T4 absorption, especially coffee, fibres and soya. Food, if present, significantly reduces T4 absorption, especially in the duodenum. For this reason, patients are advised to fast for at least 30 minutes after taking the medicament. It has been demonstrated that if T4 is taken strictly after fasting, the daily dose can be significantly reduced.

The use of numerous medicaments can also interfere with the blood levels of T4.

All malabsorption conditions, such as *coeliac disease, lactose intolerance, chronic inflammatory bowel disease and parasitosis,* can also reduce the absorption of T4 when taken orally.

In the T4 absorption process, even if active ingredient is the same, the various preparations can differ in terms of excipients and manufacturing processes liable to influence their pharmacokinetics. EP 1291021 describes T4 formulations in the form of soft or hard gelatin capsules containing a solution of active ingredient in various solvents (water, ethanol, polyethylene glycols, glycerin and mixtures thereof).

A recent innovation is the formulation of T4 in liquid oral form. As the active ingredient, which is already in solution, has no need of the gastric dissolution stage, the medicament is absorbed much more quickly. This leads to a significant reduction in waiting times between taking the medicament and having breakfast, because by the time the food reaches the small intestine, most of the liquid T4 has already been absorbed.

Liquid formulations of T4 undoubtedly improve compliance with the treatment by patients. Finally, the use of the liquid formulation of T4 can be useful in particular categories of patients unable to take the tablets (dysphagic individuals, children with an aversion to tablets, intolerance of the excipients, or patients fed through a nasogastric tube). WO 2013/030072304 and WO 2010/086030 describe solutions of T4 in mixtures of ethanol and water in single-dose containers made of suitable materials which guarantee precise control of the delivery of the solution.

The European and US regulatory authorities recently identified a series of problems associated with the actual bioavailability of the dose administered, which depends on the type of formulation and the method of administration, attributing the failure of a significant number of treatments to such causes. As the pharmacological effect is closely dependent on the dose actually absorbed, it is critical for regulatory purposes to overcome these problems. Levans, R et al., Physicochemical Basis for the Performance of Oral Solid Dosage Forms of Levothyroxine Sodium - American Association of Pharmaceutical Scientists Annual Meeting and Exposition, November 8-12, 2009, Los Angeles, California, USA) raised the problem of aggregation of the T4 molecules in an aqueous medium, which may reduce their bioavailability in practice. In fact, although T4 is considered to be a Class I compound in the Biopharmaceutics Classification System (BCS Class I compound, medicaments with high solubility and intestinal permeability), the molecule has an extremely slow intrinsic dissolution rate (IDR) of about 0.0002 mg/min/cm² in the entire physiological pH range. Thus this complex behaviour in water by T4, and its low IDR value, may explain the high failure rate of T4-based treatments due to the unpredictable, inconsistent bioavailability of the molecule.

As T4 is one of the most widely sold medicaments in the world, there is a great deal of interest in developing new oral formulations that do not prejudice the full bioavailability of the active ingredient in aqueous media. The invention described below is designed to provide a solution to these problems.

### Description of the invention

It has surprisingly been found that solubilising T4 in polyethylene glycols produces pharmaceutical formulations which, in an aqueous medium, reach concentrations far exceeding the low water-solubility value of T4, without the formation of a precipitate basically consisting of insoluble, non-bioavailable microaggregates.

In particular, it has been found that polyethylene glycols with different degrees of polymerisation (ranging from 4 to 200 monomer units), either alone or mixed with water, ethanol or glycerol, provide solvent systems for T4 which are useful for liquid oral pharmaceutical formulations, enabling concentrations of active ingredient far higher than the solubility of T4, which is only 0.105 mg/L at 25°C, to be reached in aqueous solution. This is possible because the molecules of T4 solvated in PEG, when placed in an aqueous medium, as in the simulation of a digestive process or in gastric absorption *in vivo,* give rise to the formation of soluble micellar nanoaggregates which are easily absorbed by the gastrointestinal apparatus.

The subject of the invention is therefore oral pharmaceutical formulations in drinkable liquid form comprising micellar nanoaggregates of dimensions ranging from 50 to 200 nm of tetraiodothyronine (T4) in a solvent consisting of polyethylene glycols (PEG) in percentages by weight ranging from 50 to 100% of solvent and glycerol, water, ethanol or mixtures thereof in percentages ranging from 0 to 60% by weight of solvent, preferably from 0 to 50%.

The formulations according to the invention preferably comprise solvents consisting of polyethylene glycols which are liquid at room temperature or mixtures comprising at least 50% by weight of polyethylene glycols with an average degree of polymerisation ranging from 8 to 160, and up to 50% by weight of a solvent selected from water, ethanol or glycerol, or of an 80/20 glycerol/ethanol mixture by weight.

The PEGs usable according to the invention can be liquid, with a number of monomer units ranging from 2 to 12, semi-solid, with a number of monomer units ranging from 13 to 32, or solid at room temperature, with a number of monomer units greater than 32. In the first case, the PEGs can be used as the only solvent of T4, whereas in the other cases, the PEGs are combined with water, ethanol, glycerol or mixtures thereof, preferably with water, in percentages ranging from 40 and 60% by weight.

Formulations wherein the solvent consists solely of polyethylene glycols, more preferably polyethylene glycols 200, 300, 400 or 600, are preferred. In particular, polyethylene glycol 200 having an average degree of polymerisation of 4 is preferred.

The unit doses of T4 in the formulations according to the invention can range from about 10 to about 200 µg, while the concentrations of the solutions containing PEG can range from about 10 to 250 µg/ml.

The formulations according to the invention take the form of drinkable liquid solutions, preferably in single-dose containers as described, for example, in WO2010086030 and WO2013072304.
It has been experimentally verified that at the concentrations/quantities used in pharmaceutical formulations, T4 molecules solvated in PEG do not give rise to saturation processes with formation of a microsized precipitate, and solutions of up to 0.1 g/mL can be used without the system becoming saturated. It is evident that the excellent solvating capacity (solvent-T4 interaction) is more effective than interactions driven by the hydrophobic component of T4 (T4-T4 interactions mainly based on π interactions between the aromatic rings of the active ingredient) which, in strongly polar solvents like water, are responsible for the formation of insoluble, non-bioavailable microaggregates. Surprisingly, solutions of T4 in PEG diluted even over 100 times with water at different pHs, and therefore under conditions similar to the physiological conditions that arise after the medicament is taken, do not give rise to the formation of insoluble microaggregates of T4. The solvating effect of PEG leads to aggregation of the T4 molecules to form soluble nanoaggregates with a micellar structure, having dimensions of hundreds of nanometres and containing millions of molecules. Due to their structure, said nanoaggregates are easily absorbed at gastrointestinal level, and are therefore completely and certainly bioavailable.

The following examples describe the invention in more detail.

### COMPARATIVE EXAMPLE - Determination of the quantity of aggregates formed in water by solubilising powdered crystalline T4: evaluation over time.

To establish the formation of microaggregates of T4 in an aqueous medium a fine T4 powder is dissolved in 10 mL of water (HPW), pH 6.5, at the concentration of 150 µg/mL, and the mixture is left under stirring for 5 h in an inert atmosphere. 1 L of water at pH 6.5 is then added, and the mixture is left under magnetic stirring at 600 rpm under nitrogen for 8 h, 100 mL samples being taken at intervals. 50 mL of each sample taken is microfiltered through a Millipore GSWP02500 membrane with a porosity of 0.2 µm. The concentration of T4 in the samples is then determined before and after microfiltration by HPLC (Beckman Coulter; Supelco Discovery Cyano column 100 × 2.1 mm, 5 µm, flow rate 0.2 mL/min; λ 225nm; isocratic elution H₂O/acetonitrile (80/20 w/w) + 0.1% formic acid; inj. vol. 20 µL; temperature 35°C; column pressure 130-170 Kg/cm²; run time 25 min).

At T₀ (30 sec. after mixing) the concentration of T4 in the solution is 97.3% of the theoretical value, whereas after microfiltration it is only 18.5%, indicating that T4 exists in solution mainly in the form of insoluble microaggregates, which are retained by a filter with 0.2 µm diameter pores. The T4 concentration is 97.9% at T₁ₕ, and 27.1 after microfiltration. The T4 concentration is 98.2% at T₄ₕ, and 27.5 after microfiltration. The T4 concentration is 97.5% at T₈ₕ, and 23.7 after microfiltration. Scanning electron microscopy (SEM) of the surface of the filter membranes used reveals a significant presence of elongated crystalline structures with an average length on the longer axis exceeding 3.03 ± 1.40 µm. These findings indicate that despite the 1:100 dilution process undergone by the original 150 µg/mL mixture, the formation of microaggregates remains stable, even after 8 h, and removes a considerable amount of the active ingredient from the pharmacological action.

### EXAMPLE 2 - Determination of quantity of aggregates formed in water by adding liquid formulations of T4 containing different types of liquid PEGs.

To establish whether the formation of T4 in an aqueous medium can be affected by pre-solubilisation in organic solvents compatible with pharmaceutical use, a fine T4 powder is dissolved in 10 mL of pharmaceutical grade PEG 200 or PEG 300 or PEG 400 or PEG 600 (polyethylene glycols which are liquid at room temperature, with an average degree of polymerisation of 4, 6, 8 and 12 monomer units respectively) at the concentration of 150 µg/mL, and left under stirring, under nitrogen, until the solution is completely clear. 8 mL of the different solutions of T4 in PEG is added to 800 mL of water at pH 6.5, and the mixture is left under magnetic stirring at 600 rpm, under nitrogen, for 8 h, 100 mL samples being taken at intervals. 50 mL of each sample taken is microfiltered through a Millipore GSWP02500 membrane with a porosity of 0. µm. The concentration of T4 in the samples is then determined before and after microfiltration by HPLC, as reported in example 1.

For all types of PEG used to prepare the T4 solution, at T₀ (30 sec. after mixing) the concentration of T4 in the solution before and after microfiltration is 100% of the theoretical value, indicating that T4, pre-solubilised in said PEGs which are liquid at room temperature, after 100 times dilution in water, does not give rise to the formation of insoluble microaggregates which can be retained by filters having pores with a diameter of 0.22 µm. At T₁ₕ, T₄ₕ and T₈ₕ the behaviour is similar to that at T₀, indicating that even in long times, at a stage wherein water constitutes about 99% in volume, the T4 micro-aggregation process, with formation of an insoluble product, does not take place. The same samples, filtered through membranes with a cut-off ≤ 100KDa (pores < 10-15 nm), present a quantitative absence of T4 in the permeate, indicating that T4 is present in solution in the form of completely soluble nanoaggregates. The size of said objects was determined with light scattering measurements, using the Malvern NanoSight apparatus (Nanoparticle Tracking Analysis). The soluble nanostructures have dimensions ranging from 50 to 200 nm, and as a whole consist of 5 × 10⁵ to 5 × 10⁶ molecules.

### EXAMPLE 3 - Determination of the quantity of aggregates formed in water by adding liquid formulations of T4 to aqueous solutions of PEGs with different molecular weights.

To establish whether the same preventive effect against the formation of insoluble microaggregates as described in example 2 can be obtained by using aqueous solutions of PEG, solutions in water (HPW), pH 6.5, at different concentrations (30 and 50% w/w) of pharmaceutical grade PEG 400 or PEG 2000 or PEG 4000 or PEG 8000 (polyethylene glycols with an average degree of polymerisation of 8, 40, 80 and 160 monomer units respectively) are prepared. A fine T4 powder is dissolved in 10 mL of said PEG solutions at the concentration of 150 µg/mL and left under stirring, under nitrogen, until the solution is completely clear. 8 mL of the various solutions of T4 in PEG is added to 800 mL of water at pH 6.5, and the mixture is left under magnetic stirring at 600 rpm, under nitrogen, for 8 h, 100 mL samples being taken at intervals. 50 mL of each sample taken is microfiltered through a Millipore GSWP02500 membrane with a porosity of 0. µm. The concentration of T4 in the samples is then determined before and after microfiltration by HPLC, as reported in example 1.

Solutions of T4 in 50% w/w PEG, for all types of PEG used, at T₀ (30 sec. after 100 times dilution with water) present a T4 concentration, before and after microfiltration, which is 100% of the theoretical value, indicating that T4 presolubilised in aqueous solutions of PEG wherein said polymer has concentrations exceeding 50% w/w, after 100 times dilution in water, does not give rise to the formation of insoluble microaggregates which can be retained by filters with pores having a diameter of 0.22 µm. At T₁ₕ, T₄ₕ and T₈ₕ the behaviour is similar to that at T₀, indicating that even in long times, at a stage wherein water constitutes about 99% in volume, the T4 micro-aggregation process does not take place.

However, T4 solutions in 30% w/w PEG, for all types of PEG used, at T₀ (30 sec. after 100 times dilution with water) present a T4 concentration, after microfiltration, which is 75% of the value before microfiltration, indicating that T4 pre-solubilised in aqueous solutions of PEG lower than 30% w/w, after 100 times dilution in water, gives rise to (albeit modest) formation of insoluble microaggregates. At T₁ₕ, T₄ₕ and T₈ₕ, behaviour similar to that at T₀ is observed. The samples obtained by using 50% PEG in water, filtered through membranes with a cut-off ≤ 100 KDa (pores < 10-15 nm) present a quantitative absence of T4 in the permeate, indicating that T4 is present in solution in the form of completely soluble micellar nanoaggregates. The size of said objects was determined with light scattering measurements, using the Malvern NanoSight apparatus (Nanoparticle Tracking Analysis). The soluble nanostructures have dimensions ranging from 50 to 200 nm, and as a whole consist of 5 × 10⁵ to 5 × 10⁶ molecules.

### Example 4 - Effect of pH on formation of T4 aggregates in an aqueous medium.

To establish whether the same preventive effect against the formation of insoluble, non-bioavailable microaggregates as described in example 3 can be obtained in an acid medium by simulating a gastric environment *in vitro* using aqueous solutions of PEG, solutions in water (HPW), pH 6.5, containing 50% w/w of pharmaceutical grade PEG 400 or PEG 2000 or PEG 4000 or PEG 8000 (polyethylene glycols with an average degree of polymerisation of 8, 40, 80 and 160 respectively) are prepared. A fine T4 powder is dissolved in 10 mL of said PEG solutions at the concentration of 150 µg/mL, and left under stirring until the solution is completely clear. 8 mL of the various solutions of T4 in PEG is added to 800 mL of water at pH 1.5, and the mixture is left under magnetic stirring at 600 rpm, under nitrogen, for 8 h, 100 mL samples being taken at intervals. 50 mL of each sample taken is microfiltered through a Millipore GSWP02500 membrane with a porosity of 0.2 µm. The concentration of T4 in the samples is then determined before and after microfiltration by HPLC, as reported in example 1.

**Solutions of T4 in 50% w/w PEG, for all types of PEG used, a**t T₀ (30 sec. after 100 times dilution with water at pH 1.5) present a T4 concentration, before and after microfiltration, which is 100% of the theoretical value, indicating that T4 presolubilised in aqueous solutions of PEG exceeding 50%, after 100 times dilution in water at pH 1.5, **does not give rise to the formation of insoluble microaggregates** which can be retained by filters with pores having a diameter of 0.22 µm. At T₁ₕ, T₄ₕ and T₈ₕ the behaviour is similar to that at T₀, indicating that even in long times, in a strongly acid medium, at a stage wherein water constitutes about 99% in volume, the T4 microaggregation process that forms insoluble, non-bioavailable microaggregates does not take place. The same samples, filtered through membranes with a cut-off ≤ 100 KDa (pores < 10-15 nm), present a quantitative absence of T4 in the permeate, indicating that T4 is present in solution solely in the form of nanoaggregates which are completely soluble and bioavailable. The size of said objects was determined with light scattering measurements, using the Malvern NanoSight apparatus (Nanoparticle Tracking Analysis). The soluble nanostructures have dimensions ranging from 50 to 200 nm, and as a whole consist of 5 × 10⁵ to 5 × 10⁶ molecules.

This result as a whole is of considerable importance, confirming that the acid medium, at gastric level, leaves the ability of PEG to prevent the formation of insoluble microaggregates of T4 unchanged, while the ability of T4 to organise itself into easily absorbable micellar nanostructures remains unchanged.

### Example 5 - Determination of quantity of aggregates formed in water by adding liquid formulations of T4 to mixtures of PEG with glycerol or ethanol.

To establish whether the same effect of preventing the formation of insoluble, non-bioavailable microaggregates as reported in examples 2-4 can be obtained with mixtures of PEG and glycerol or ethanol as T4 solvent system, solutions of T4 in PEG 8000/glycerol and in PEG/ethanol 50/50 w/w are prepared. A fine T4 powder is dissolved in 10 mL of said solvent mixtures at the concentration of 150 µg/mL, and left under stirring until the solution is completely clear. 8 mL of the two solutions of T4 in PEG is added to 800 mL of water at pH 6.5, and the mixture is left under magnetic stirring at 600 rpm, under nitrogen, for 8 h, 100 mL samples being taken at intervals. 50 mL of each sample taken is microfiltered through a Millipore GSWP02500 membrane with a porosity of 0.2 µm. The concentration of T4 in the samples is then determined before and after microfiltration by HPLC, as reported in example 1.

For both solvent systems tested, at T₀ (30 sec. after mixing) the T4 concentration after microfiltration is identical to that before microfiltration, indicating that T4, pre-solubilised in said solvents at room temperature, after 100 times dilution in water, does not give rise to the formation of insoluble, non-bioavailable microaggregates. At T₁ₕ, T₄ₕ and T₈ₕ the behaviour is similar to that at T₀, indicating that even in long times, at a stage wherein water constitutes about 99% in volume, the T4 micro-aggregation process does not take place.

The same samples, filtered through membranes with a cut-off ≤ 100 KDa (pores < 10-15 nanometres) present a quantitative absence of T4 in the permeate, indicating that T4 is present in solution solely in the form of nanoaggregates which are completely soluble. The size of said objects was determined with light scattering measurements, using the Malvern NanoSight apparatus (Nanoparticle Tracking Analysis). The soluble nanostructures have dimensions ranging from 50 to 200 nm, and as a whole consist of 5 × 10⁵ to 5 × 10⁶ molecules.

### Example 6 - Determination of quantity of aggregates formed in water by adding liquid formulations of T4 to different solvent systems.

To establish whether the same effect of preventing the formation of insoluble, non-bioavailable microaggregates as reported in examples 2-5 can be obtained with organic solvents other than PEG, solutions of T4 in glycerol, ethanol and mixtures thereof are prepared. A fine T4 powder is dissolved in 10 mL of glycerin or ethanol or glycerin/ethanol 80/20 w/w (pharmaceutical grade) at the concentration of 150 µg/mL and left under stirring until the solution becomes completely clear. 8 mL of the various solutions of T4 in PEG is added to 800 mL of water at pH 6.5, and the mixture is left under magnetic stirring at 600 rpm, under nitrogen, for 8 h, 100 mL samples being taken at intervals. 50 mL of each sample taken is microfiltered through a Millipore GSWP02500 membrane with a porosity of 0.2 µm. The T4 concentration in the samples is then determined before and after microfiltration by HPLC, as reported in example 1.

For all the solvent systems tested, at T₀ (30 sec. after mixing) the T4 concentration after microfiltration ranges from 90 to 95% of the pre-microfiltration value, indicating that T4, pre-solubilised in said solvents at room temperature, after 100 times dilution in water, gives rise to the formation of insoluble, non-bioavailable microaggregates, but amounting to 5-10% of the dose, thus making said formulations compatible with clinical use. At T₁ₕ, T₄ₕ and T₈ₕ the behaviour is similar to that at T₀, indicating that even in long times, at a stage wherein water constitutes about 99% in volume, the T4 micro-aggregation process is very low.

The same samples, filtered through membranes with a cut-off ≤ 100 KDa (pores < 10-15 nanometres), present a quantitative absence of T4 in the permeate, indicating that T4 is present in solution in the form of completely soluble micellar nanoaggregates. The size of said objects was determined with light scattering measurements, using the Malvern NanoSight apparatus (Nanoparticle Tracking Analysis). The soluble nanostructures have dimensions ranging from 50 to 200 nm, and as a whole consist of 5 × 10⁵ to 5 × 10⁶ molecules. This demonstrates the possibility of also using solvent systems other than PEG for T4, although with slightly inferior results.

### Example 7 - Protective effect of PEG

A fine T4 powder at the concentration of 150 µg/mL is dissolved in 10 mL of the following solvents: ethanol, glycerol, PEG 200, PEG 300, PEG 400 and PEG 600. 5 mL of each solution is left under stirring for 10 h at 600 rpm and 35°C in a nitrogen atmosphere, and 5 mL is left under the same conditions but in an oxygen atmosphere. The T4 content is then determined by HPLC, as described in example 1. The assay value of the solutions under nitrogen in all cases remains the same as the theoretical value, whereas for solutions in an oxygen atmosphere, those which have glycerol and ethanol as solvent present a residual T4 assay value which is only 30% of the original assay value, and those which have PEG as solvent present an assay value ranging from 80 to 90% of the original assay value. This demonstrates that PEG has a strong protective action against the oxidative degradation processes of T4, indicating the existence of advantages for the half-life of pharmaceutical formulations made with this type of solvent.

## Claims

1. Liquid oral pharmaceutical formulations comprising micellar nanoaggregates of dimensions ranging from 50 to 200 nm of tetraiodothyronine in a solvent consisting of polyethylene glycols in percentages by weight ranging from 50 to 100% of the solvent, and glycerol, water, ethanol or mixtures thereof in percentages by weight ranging from 0 to 60% of the solvent.

2. Liquid formulations according to claim 1 wherein the percentages by weight of glycerol, water and ethanol range from 0 to 50%.

3. Liquid formulations according to claim 1 or 2 wherein the solvent consists of one or more polyethylene glycols with a number of monomeric units ranging from 4 to 12.

4. Liquid formulations according to one or more of claims 1 to 2 wherein the solvent consists of PEG with a number of monomer units ranging from 13 and 32 in a mixture with water, ethanol, glycerol or mixtures thereof.

## Patentansprüche

1. Flüssige orale pharmazeutische Zubereitungen, umfassend mizelläre Nanoaggregate von Tetraiodthyronin mit Abmessungen im Bereich von 50 bis 200 nm in einem Lösungsmittel, das aus Polyethylenglycolen besteht, in Gewichtsprozentanteilen im Bereich von 50 bis 100 Gew.-% des Lösungsmittels und Glycerin, Wasser, Ethanol oder Gemische davon in Gewichtsprozentanteilen im Bereich von 0 bis 60 Gew.-% des Lösungsmittels.

2. Flüssige Zubereitungen gemäß Anspruch 1, wobei die Gewichtsprozentanteile von Glycerin, Wasser und Ethanol im Bereich von 0 bis 50 Gew.-% liegen.

3. Flüssige Zubereitungen gemäß Anspruch 1 oder 2, wobei das Lösungsmittel aus einem oder mehreren Polyethylenglycolen mit einer Anzahl von monomeren Einheiten im Bereich von 4 bis 12 besteht.

4. Flüssige Zubereitungen gemäß einem oder mehreren der Ansprüche 1 bis 2, wobei das Lösungsmittel aus PEG mit einer Anzahl von Monomereinheiten im Bereich von 13 bis 32 in einem Gemisch mit Wasser, Ethanol, Glycerin oder Gemischen davon besteht.

## Revendications

1. Formulations pharmaceutiques liquides à administration par voie orale, comprenant des nano-agrégats micellaires dont les dimensions se situent dans une plage de 50 à 200 nm de tétraiodothyronine dans un solvant constitué par des polyéthylèneglycols dans des pourcentages en poids qui se situent dans une plage de 50 à 100 % du solvant, et par du glycérol, de l'eau, de l'éthanol ou leurs mélanges dans des pourcentages en poids qui représentent de 0 à 60 % du solvant.

2. Formulations liquides selon la revendication 1, dans lesquelles les pourcentages en poids du glycérol, de l'eau et de l'éthanol se situent dans une plage de 0 à 50 %.

3. Formulations liquides selon la revendication 1 ou 2, dans lesquelles le solvant est constitué par un ou plusieurs polyéthylèneglycols dont le nombre d'unités monomères se situe dans une plage de 4 à 12.

4. Formulations liquides selon une ou plusieurs des revendications 1 à 2, dans lesquelles le solvant est constitué de PEG dont le nombre d'unités monomères se situe dans une plage de 13 à 32 dans un mélange avec de l'eau, de l'éthanol, du glycérol ou leurs mélanges.
